# EUROPEAN PATENT APPLICATION

(11) **EP 2 025 266 A1**
(43) Date of publication of application: **18.02.2009**
(21) Application number: 07742761.5
(22) Date of filing: 01.05.2007
(51) Int. Cl.: A47G 9/10, H04R 1/00, H04R 1/02

(54) **PILLOW DEVICE**

(30) Priority: 08.06.2006 JP 2006160140; 28.02.2007 JP 2007050087
(71) Applicant: France Bed Co., Ltd., Akishima-shi Tokyo 196-0022 (JP)
(72) Inventor: OGUMA, Yoshio, Tokyo (JP); HARADA, Yoshikazu, Tokyo (JP)
(74) Representative: Rehmann, Thorsten
(86) International application number: PCT/JP2007/059326
(87) International publication number: WO 2007/141975

(57) **Abstract**

A pillow device which supports a head portion of a user during sleep includes a pillow main body (2), a microphone (9), which is provided in the pillow main body and detects a snore of the user, a vibrator (11), which operates in response to the snore detected by the microphone and moves the pillow main body, a pressure detecting switch (12), which is provided in the pillow main body and detects a change in pressure exerted on the pillow main body when the user moves the head portion, and a gate circuit (27), which inhibits the vibrator from operating for a predetermined period of time, when the pressure detecting switch detects a change in pressure exerted on the pillow main body.

## Description

### Technical Field

The present invention relates to a pillow device to prevent the user from snoring while sleeping.

### Background Art

While the user is sleeping on a pillow, he or she may snore depending on the height or hardness of the pillow, or the lying posture. If the snore is loud, others around the user may feel unpleasant or the user may awake by his or her snore. Thus, the loud snore is undesirable.

A technique to stop snoring is known, in which when the user snores during sleep, the snore is detected by a microphone, and, for example, a vibrator incorporated in a pillow is activated upon detection of the sound to give a slight shock to the user.

Patent Publication 1 discloses that when a snore is detected by a microphone, a vibrator incorporated in a pillow is activated upon detection of the sound to give vibrations to the head of the user, thereby stopping snoring.

Patent Publication 1: Jpn. Pat. Appln. KOKAI Publication No. 2004-283381

If the microphone is configured to detect a snore, it may also detect rustling of a comforter or the like or the pillow that occurs when the user turns over. In other words, the microphone may detect a sound other than snores.

According to the conventional art, however, when the microphone detects a sound other than snores, a vibrator is activated without distinguishing the sound from snores. Therefore, even if the user is not snoring, the vibrator may move the head of the user. In this case, the user's sleep is interrupted, which is practically undesirable.

### Disclosure of Invention

The present invention has been made provide a pillow device, which prevents the head of the user supported by the pillow from moving when a sound other than snores is generated because, for example, the user turns over.

According to the present invention, therefore, there is provided a pillow device which supports a head portion of a user during sleep, the device comprising:
a pillow main body;
a microphone, which is provided in the pillow main body and detects a snore of the user and other sounds;
driving means, which operates when the microphone detects a snore, for driving the pillow main body;
detecting means, provided in the pillow main body, for detecting a change in pressure exerted on the pillow main body when the user moves the head portion; and
inhibiting means for inhibiting the driving means from operating for a predetermined period of time even if the microphone detects a sound other than a snore, when the detecting means detects a change in pressure exerted on the pillow main body.

### Brief Description of Drawings

FIG. 1 is a perspective view of a pillow according to a first embodiment of the present invention, showing a state in which a part of an exterior cover is cut away.
FIG. 2 is a schematic perspective view showing a control device, microphones, pressure detecting switches and a vibrator.
FIG. 3 is a block diagram of a control circuit provided in the control device.
FIG. 4 is a block diagram of a control circuit according to a second embodiment of the present invention.
FIG. 5 is a perspective view of a pillow according to a third embodiment of the present invention, showing a state in which a part of an exterior cover is cut away.

### Best Mode for Carrying Out the Invention

Embodiments of the present invention will be described with reference to the drawings.

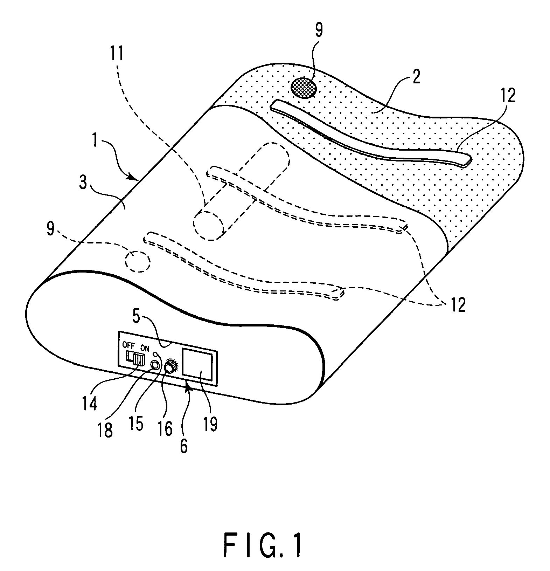

FIGS. 1 to 3 show a first embodiment of the present invention. A pillow 1 shown in FIG. 1 has a pillow main body 2 formed by molding an elastic material, for example a urethane foam. The pillow main body 2 is covered by an exterior cover 3 sewn into a bag shape.

A side surface of the pillow 1 is opened, and a storage portion 5 is formed in the opening. A control device 6 is removably stored in the storage portion 5. The inner surface of the storage portion 5 is covered with the exterior cover 3.

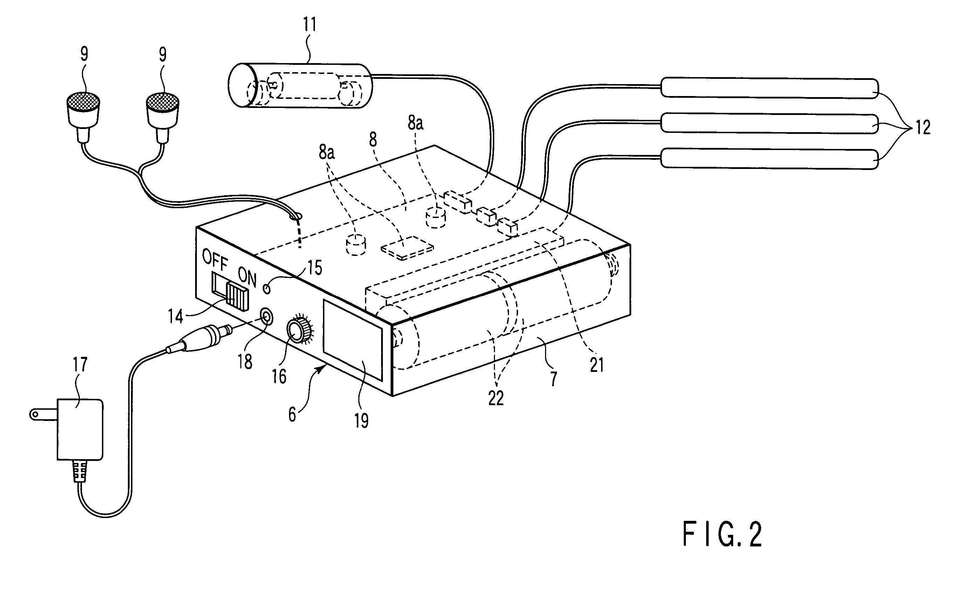

The control device 6 has a case 7 as shown in FIG. 2. The case 7 contains a circuit board 8, on which a control circuit composed of electronic parts 8a is mounted. The circuit board 8 is electrically connected to a pair of capacitor microphones 9, a vibrator 11 as driving means for moving the pillow main body 2, and three flexible tape-like pressure detecting switches 12 which detect a pressure exerted on the upper surface of the pillow main body 2.

On one side surface of the case 7, there are provided a power supply switch 14, a power supply lamp 15, a sensitivity adjustment volume 16, and a connecting portion 18 to which an AC adapter 17 is connected. Further, a lid 19, which can be opened and closed, is provided on the side of the case 7. When the lid 19 is opened, a recorder 21, such as an IC recorder, and a battery 22 can be inserted in and removed out of the case 7.

As shown in FIG. 1, the microphones 9 are exposed through the upper surface of the pillow main body 2 at end portions in the width direction of the pillow main body 2 and an end portion in the fore-and-aft direction thereof. The three pressure detecting switches 12 are immovably fixed, for example, adhered, to the upper surface of the pillow main body 2 at regular intervals along the fore-and-aft direction crossing the width direction of the pillow main body 2, as shown in FIG. 1. The vibrator 11 is buried in the end portion in the fore-and-aft direction in a central portion in the width direction.

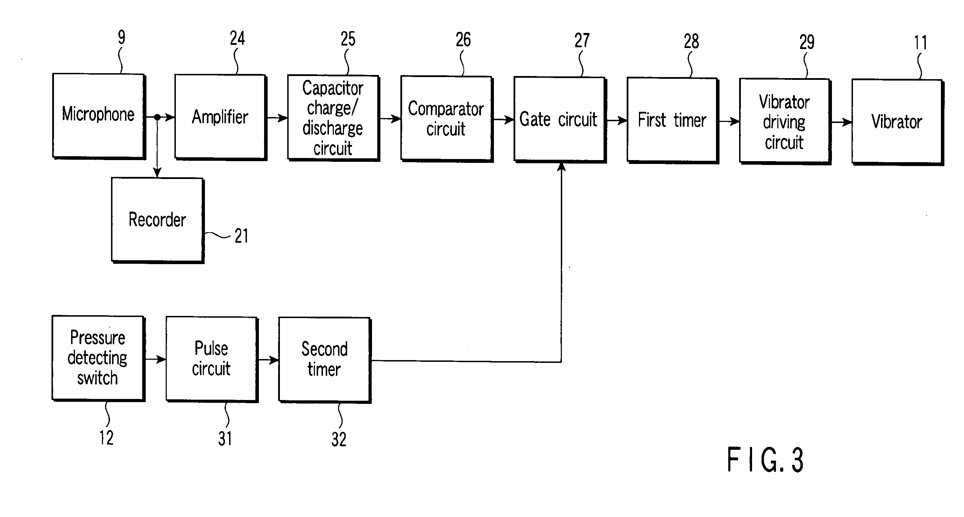

FIG. 3 is a block diagram of the control circuit mounted on the circuit board 8. A sound detected by the microphones 9 is output to an amplifier 24 and a recorder 21. The recorder 21 records the sound detected by the microphones 9, and the amplifier 24 amplifies a sound signal output from the microphones 9.

The sound signal amplified by the amplifier 24 is charged by a capacitor charge/discharge circuit 25. The capacitor charge/discharge circuit 25 is charged, taking the magnitude or continuity of the sound signal into account. It has a discharge resistor (not shown), and is gradually discharged when not charged.

The value of a voltage charged by the capacitor charge/discharge circuit 25 is compared with a set value set in a comparator circuit 26. If the value of charged voltage is higher than the set value, the comparator circuit 26 outputs a pulse signal to a gate circuit 27.

The pulse signal output from the comparator circuit 26 generally passes through the gate circuit 27, and is input to a first timer 28 and operates it. The operation time of the first timer 28 is set to a short period of time, for example, one second. The first timer 28 causes a signal to be output to a vibrator driving circuit 29 for the set time. As a result, the vibrator 11 in the pillow main body 2 operates for one second.

When the pressure distribution state on the upper surface of the pillow main body 2 changes, at least one of the three pressure detecting switches 12 is activated due to the change from an unpressurized state to a pressurized state. For example, if the user using the pillow 1 turns over and moves sideways the head supported in a central portion in the width direction of the pillow main body 2, one of the pressure detecting switches 12, which is located at one end portion in the width direction, is operated and detects a change in the pressure exerted on the upper surface of the pillow main body 2.

When at least one of the pressure detecting switches 12 detects a change in pressure, it outputs a detection signal, which is input to a pulse circuit 31. Accordingly, the pulse circuit 31 outputs a pulse signal, which is input to a second timer 32. The second timer 32, which receives the pulse signal, outputs an output inhibition signal to the gate circuit 27 for a predetermined set period of time. The period in which the second timer 32 outputs the output inhibition signal, that is, the set period of the second timer 32, is set longer than that of the first timer 28. In this embodiment, the set period is 15 seconds.

While the output inhibition signal of the second timer 32 is being input to the gate circuit 27, the pulse signal from the comparator circuit 26 is inhibited from being input to the first timer 28 via the gate circuit 27. As a result, during the time when the second timer 32 is operating, even if the microphones 9 detect a sound and a pulse signal generated by the comparator circuit 26 is input to the gate circuit 27, the pulse signal cannot be output to the first timer 28. Therefore, the vibrator 11 cannot be activated.

With the above constitution, when the user sleeping with the head supported by the pillow 1 snores, the microphones 9 detect the snore. The electric signal of the snore detected by the microphones 9 is amplified by the amplifier 24 and charged by the capacitor charging/discharging circuit 25. The value of the charged voltage is compared with the set value of the comparator circuit 26.

If the value of the charged voltage of the capacitor charging/discharging circuit 25 is greater than the set value of the comparator circuit 26, the comparator circuit 26 outputs a pulse signal to the gate circuit 27, so that the first timer 28 is operated for one second. As a result, the vibrator 11 is driven for one second and gives vibrations to the head of the user supported on the pillow 1. Thus, the snore of the user is stopped by the stimulation of the vibrations.

When the user turns over during sleep, even if a comforter or the like or the pillow rustles and causes a noise other than snores, the microphones 9 may detect the noises. For example, when the user moves the head from the central portion to one end portion in the width direction of the pillow 1, one of the three pressure detecting switches 12 provided on the upper surface of the pillow main body 2, which is located in the one end portion in the width direction of the pillow 1, is changed from the unpressurized state to the pressurized state. Then, the pressurized pressure detecting switch is activated.

When at least one of the three pressure detecting switches 12 detects a change in pressure by the user's head exerted on the upper surface of the pillow main body 2, the pulse circuit 31 outputs a pulse signal to the second timer 32. As a result, the second timer 32 operates for 15 seconds.

While the second timer 32 is operating, even if the microphones 9 detect a noise and the comparator circuit 26 outputs a pulse signal to the gate circuit 27, the pulse signal is shut off by the gate circuit 27. Therefore, the vibrator 11 cannot be activated.

Thus, even if the microphones 9 detect a noise other than snores when the user turns over, the vibrator 11 is not operated by the noise and accordingly does not stimulate the head of the user. In other words, the user, who is not snoring, is not awaked unexpectedly due to stimulation by the vibrator 11.

The snores or noises detected by the microphones 9 are recorded by the recorder 21. Therefore, it is possible to have the recorded snores diagnosed by a medical specialist to determine whether or not the snoring of the user is an abnormal one caused by, for example, the sleep apnea syndrome.

The storage portion 5 is opened at a side surface of the pillow 1, and stores the control device 6. Thus, since the pillow 1 is integral with the control device 6, it is easy to handle. Moreover, since the control device 6 can be operated by either the AC adapter 17 or the battery 22, the pillow can be used in any place.

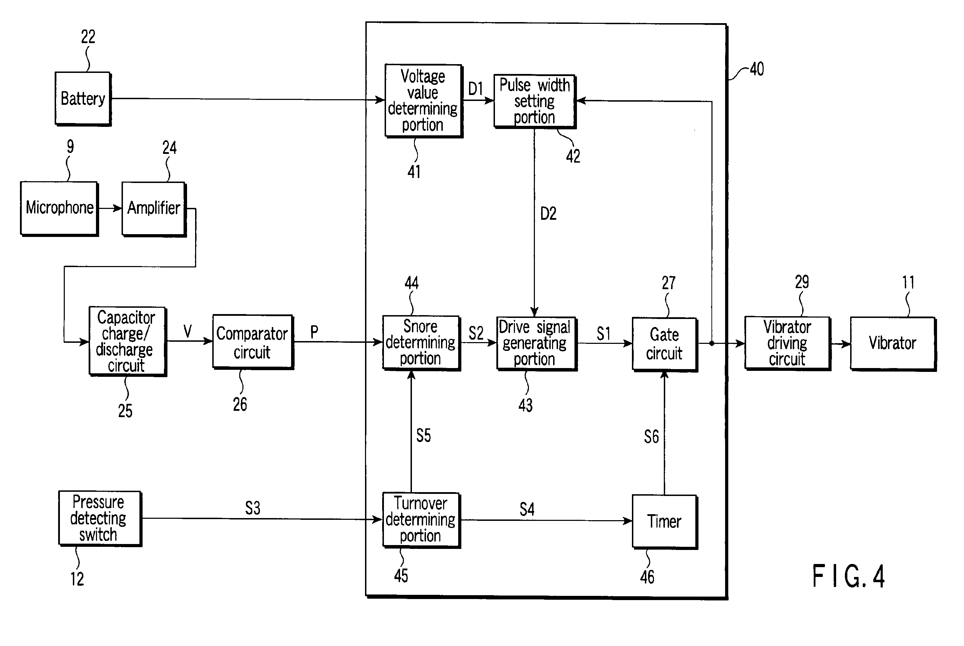

FIG. 4 shows a second embodiment of the present invention. In FIG. 4, the same parts as those in the first embodiment shown in FIG. 3 are identified by the same reference numerals and the descriptions thereof are omitted. In the second embodiment, the vibrator 11 is driven by a battery 22. A voltage value determining portion 41 in a program processing portion 40 determines the voltage of the battery 22.

The voltage value determining portion 41 determines the voltage value of the battery 22 to be, for example, one of five levels of E1 to E5, and outputs voltage data D1 to a pulse width setting portion 42. The pulse width setting portion 42 sets three pulse widths of "large", "middle" and "small" for each of the voltage data D1 of the levels E1 to E5 as pulse width data D2. The pulse width data D2 are output to a drive signal generating portion 43.

The drive signal generating portion 43 generates a drive signal S1 in accordance with the level E1 to E5. When the voltage value of the battery 22 is lowered, the pulse width of the drive signal S1 is set to "large", "middle" or "small", depending on the level E1 to E5. Thus, the drive signal generating portion 43 is configured to output the drive signals S1 of the 15 possible pulse widths, as will be described later.

As well as in the first embodiment, the charge voltage value V is compared to the set value in the comparator circuit 26. When the charge voltage value V is higher than the set value, the comparator circuit 26 outputs a pulse signal P to the gate circuit 27.

The pulse signal P output from the comparator circuit 26 is input to a snore determining portion 44. When the snore determining portion 44 receives pulse signals P from the comparator circuit 26 a plurality of times in a predetermined time, for example, three times in 30 seconds, it determines that the sound detected by the microphones 9 is a snore and outputs a snore signal S2 to the drive signal generating portion 43. In other words, when the sound detected by the microphones 9 has periodicity, the snore determining portion 44 determines that the sound is a snore.

The drive signal generating portion 43, which has received the snore signal S2, outputs the drive signal S1 having the pulse width set by the pulse width setting portion 42 to the gate circuit 27. When one of the pressure detecting switches 12 detects a turnover of the user, it outputs a turnover signal S3 to a turnover determining portion 45. The turnover determining portion 45 outputs a start signal S4 to a timer 46 and, simultaneously, a clear signal S5 to the snore determining portion 44.

When the turnover determining portion 45 outputs the start signal S4 to the timer 46, the timer 46 outputs an output inhibition signal S6 to the gate circuit 27 for a predetermined period of time, for example, 30 seconds. Therefore, even if the drive signal generating portion 43 outputs the drive signal S1, the drive signal S1 cannot pass through the gate circuit 27 and cannot be input to the vibrator driving circuit 29.

When the turnover determining portion 45 outputs the clear signal S5 to the snore determining section 44, the determination data memory in the snore determining portion 44 is initialized.

While the pressure detecting switches 12 detect no turnover of the user, if the drive signal generating portion 43 outputs the drive signal S1, the drive signal S1 is passed through the gate circuit 27 and output to the vibrator driving circuit 29. As a result, the vibrator 11 is driven at the level corresponding to the pulse width of the drive signal S1.

The drive signal S1, which has passed through the gate circuit 27, is output as vibration output data to the vibrator driving circuit 29 and the pulse width setting portion 42. The pulse width setting portion 42 measures an interval between the time when the drive signal S1 was previously input and the time when the drive signal S1 is currently input. If the interval is a predetermined time or shorter, for example, 20 seconds or shorter, the pulse width setting portion 42 outputs to the drive signal generating portion 43 pulse width data to increase the pulse width of the drive signal S1 output from the drive signal generating portion 43 greater than the previous pulse width.

As a result, the pulse width of the drive signal S1 output from the drive signal generating portion 43 is increased, so that the vibrator 11 is operated to produce vibrations at higher level than previously. More specifically, if the previous pulse width is "small", the drive signal S1 of a "middle" pulse width is output. If the previous pulse width is "middle", the drive signal S1 of a "large" pulse width is output.

In other words, if the user does not stop snoring even if the vibrator 11 is driven three times at low-level vibrations, the vibrator 11 is then driven three times to produce middle-level vibrations. If the user continues snoring, the vibrator 11 is driven three times to produce high-level vibrations.

Since the level of the vibrations of the vibrator 11 is thus increased stepwise, the snoring of the user is stopped reliably. Thus, the storing of the user can be stopped without giving much uncomfortable feeling to the user.

The pulse width of the drive signal S1 output from the drive signal generating portion 43 is also controlled, depending on the voltage value of the battery 22 measured by the voltage value determining portion 41. Specifically, the voltage value of the battery 22 is determined to be one of the five levels of E1 to E5, and if the voltage value is lowered, the pulse width is increased

Therefore, even if the voltage value is lowered because of the exhaustion of power of the battery 22, if the lowered voltage value falls within the range of E1 to E5, the vibrator 11 can produce vibrations of substantially the same level.

As described above, according to the second embodiment, when the user periodically snores, the snore determining portion 44 detects that fact. Therefore, if the user snores only once, the vibrator 11 is not driven.

The voltage value determining portion 41 detects the voltage value of the battery 22 for driving the vibrator 11, and outputs the voltage data D1. If the voltage data D1 indicates that the voltage value is lowered because of the consumption of power of the battery 22, the pulse width of the drive signal S2 is increased in accordance with the lowering of the voltage value. Therefore, even if the voltage value of the battery 22 lowers, the vibrator 11 can produce vibrations of substantially the same level.

The pulse width setting portion 42 measures the interval between the time when the drive signal S1 for driving the vibrator 11 previously passes through the gate circuit 27 and the time when the drive signal S1 currently passes. If the interval is a predetermined time or longer, the pulse width of the drive signal S1 is increased, so that the vibrator 11 is driven to increase the vibration level stepwise at three levels.

Specifically, if the user does not stop snoring even if the vibrator 11 gives low-level vibrations to the user three times, the vibrator 11 produces middle-level vibrations. If the snoring does not stop by the middle-level vibrations, the vibrator 11 produces high-level vibrations. Thus, the user can stop snoring without suddenly receiving high-level vibrations; that is, the user will not feel uncomfortable to stop snoring.

FIG. 4 shows a third embodiment of the present invention. In this embodiment, a single tape-like pressure detecting switch 12 is provided on the upper surface of the pillow main body 2 along the width direction thereof. When the user turns over and the head moves on the pillow main body 2, even the single pressure detecting switch 12 can reliably detect a change in pressure.

The second embodiment is described as an example, in which the battery is used as a power supply. However, the AC adapter can also be used as well as in the first embodiment.

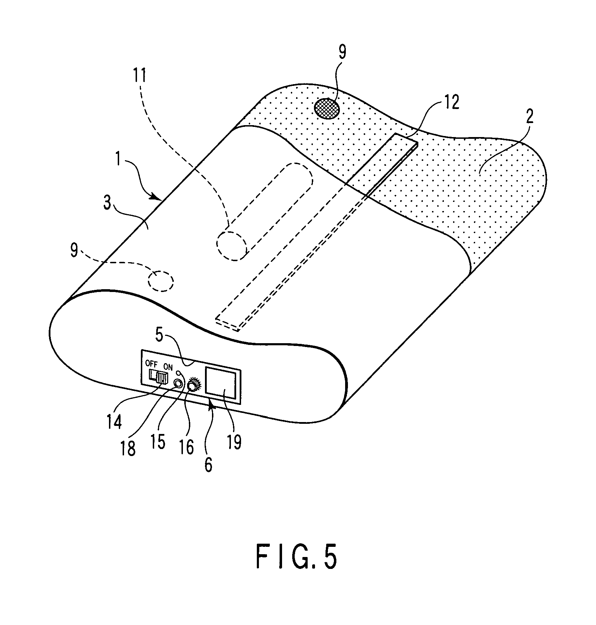

FIG. 5 shows a third embodiment of the present invention. In this embodiment, instead of the three tape-like pressure detecting switches 12 in the first and second embodiments, a single pressure detecting switch 12 is provided along the entire length in the width direction of the pillow main body 2 in a middle portion in the fore-and-aft direction thereof.

With this configuration also, if the user turns over and moves the head in the width direction of the pillow main body 2, the movement can be reliably detected by the single pressure detecting switch 12 extending along the width direction of the pillow main body 2.

In the first and second embodiments, when the microphones detect a snore, the vibrator incorporated in the pillow main body is operated to vibrate the pillow main body to stimulate the user. However, instead of using the vibrator, the pillow main body may be placed on a mount plate, which slides the pillow main body in width directions or fore-and-aft directions by means of a driver. In this case, when the microphones detect a snore, the driver may be operated to slide the pillow.

Alternatively, the mount plate may be driven up and down by the driver. In this case, when the microphones detect a snore, the driver may be operated to move the pillow up and down.

### Industrial Applicability

According to the present invention, when the user moves the head in the pillow, a change in pressure exerted on the pillow main body is detected. Due to the detection, the pillow main body is prevented from moving for a predetermined period of time, even if the microphones detect a sound.

## Claims

1. A pillow device which supports a head portion of a user during sleep, the device **characterized by** comprising:
a pillow main body;
a microphone, which is provided in the pillow main body and detects a snore of the user and other sounds;
driving means, which operates when the microphone detects a snore, for driving the pillow main body;
detecting means, provided in the pillow main body, for detecting a change in pressure exerted on the pillow main body when the user moves the head portion; and
inhibiting means for inhibiting the driving means from operating for a predetermined period of time even if the microphone detects a sound other than a snore, when the detecting means detects a change in pressure exerted on the pillow main body.

2. The pillow device according to claim 1, **characterized in that** the snore detected by the microphone is converted to an electric signal, which is compared with a reference value by a comparing portion, and the comparing portion outputs a drive signal to drive the driving means based on the comparison.

3. The pillow device according to claim 2, **characterized by** further comprising a determining portion, which is provided between the comparing portion and the driving means and determines that a sound detected by the microphone is a snore, when the drive signal based on the comparison in the comparing portion is repeatedly input a plurality of times.

4. The pillow device according to claim 2, **characterized in that**:
the drive signal is input to the driving means through a gate circuit;
a detection signal from the detecting means operates a timer, and an output inhibition signal output from the timer is input to the gate circuit; and
the gate circuit inhibits the drive signal from being output to the driving means, while the output inhibition signal output from the timer is being input to the gate circuit.

5. The pillow device according to claim 4, **characterized by** further comprising a pulse width setting portion, which measures a time interval between an output of the drive signal from the gate circuit to the driving means and an output of a previous drive signal from the gate circuit, and increases a pulse width of the drive signal to drive the driving means if the time interval is a predetermined period of time or shorter.

6. The pillow device according to claim 5, **characterized in that** the driving means is driven by a battery, the device further comprising a voltage value determining portion which measures a voltage of the battery, and when the voltage of the battery is lowered, the pulse width setting portion increases the pulse width of the drive signal to drive the driving means.

7. The pillow device according to claim 1, **characterized in that** the driving means is a vibrator provided in the pillow main body.

8. The pillow device according to claim 1, **characterized by** further comprising a recorder which records the snore of the user detected by the microphone.

9. The pillow device according to claim 1, **characterized in that** the detecting means is a tape-like pressure detecting switch provided on an upper surface of the pillow main body.

10. The pillow device according to claim 1, **characterized by** further comprising a control device including a control circuit to control driving of the driving means, the control device being stored in a storing portion opened on a side surface of the pillow main body.

11. The pillow device according to claim 8, **characterized in that** the recorder is removably contained in the control device.
